# FASCICULE DE BREVET EUROPEEN

(11) **EP 1 810 960 B1**
(45) Date de publication et mention de la délivrance du brevet: **18.11.2009**
(21) Numéro de dépôt: 07290061.6
(22) Date de dépôt: 16.01.2007
(51) Int. Cl.: C07C 67/475, C07C 67/333, C07C 51/347, C07C 69/593, C07C 69/533, C07C 6/04, C07C 11/04, C07C 11/02, C07C 57/13

(54) **Procédé de co-production d'oléfines et de diesters ou de diacides à partir de corps gras insaturés**
Verfahren zur gleichzeitigen Produktion von Olefinen und Diestern oder Disäuren aus ungesättigten Fettkörpern
Method of co-producing olefins and diesters or diacids from unsaturated fatty bodies

(30) Priorité: 24.01.2006 FR 0600646
(43) Date de publication de la demande: 25.07.2007
(73) Titulaire: Institut Français du Pétrole, 92852 Rueil Malmaison Cedex (FR)
(72) Inventeur: Olivier-Bourbigou, Hélène, 69230 Saint Genis Laval (FR); Vallee, Christophe, 38600 Fontaine (FR); Hillion, Gérard, 95220 Herblay (FR)

(56) Documents cités:
- EP-A- 1 698 686
- US-A- 5 675 051
- US-B1- 6 756 500
- WARWEL S ET AL: "Polymers and surfactants on the basis of renewable resources" CHEMOSPHERE, PERGAMON PRESS, OXFORD, GB, vol. 43, 2001, pages 39-48, XP002262684 ISSN: 0045-6535
- OLIVIER-BOURBIGOU H ET AL: "Ionic liquids: perspectives for organic and catalytic reactions" JOURNAL OF MOLECULAR CATALYSIS. A, CHEMICAL, ELSEVIER, AMSTERDAM, NL, 2002, pages 419-437, XP002274532 ISSN: 1381-1169

## Description

### Domaine de l'invention

L'invention concerne un procédé de coproduction d'oléfines et de diesters ou diacides insaturés à partir de corps gras insaturés.

### Arrière-plan technologique

Les oléfines alpha, et plus particulièrement le décène-1, sont des intermédiaires de synthèse recherchés en pétrochimie et sont habituellement entièrement fabriqués à partir de matières premières d'origine fossiles comme l'éthylène. Le décène-1 intervient notamment dans la fabrication de poly-alpha-oléfines (PAO), qui sont des lubrifiants de synthèse ainsi que dans la préparation d'alcools et dans bien d'autres procédés de fabrication en chimie industrielle. La fabrication de ces oléfines à partir d'une matière première pour l'essentiel d'origine animale ou végétale, donc renouvelable, est potentiellement d'un grand intérêt.

Les diesters ou les diacides à longues chaines, et plus particulièrement l'acide 9-octadécène-1,18-dioique, intermédiaire recherché pour la fabrication de certains polymères, sont généralement obtenus par fermentation de paraffines issues du pétrole. Leur fabrication à partir d'une matière première pour l'essentiel d'origine animale ou végétale, donc renouvelable, est également potentiellement d'un grand intérêt.

Ces deux composés peuvent être obtenus, de façon indépendante, à partir de matières premières d'origine végétale ou animale, par exemple à partir d'acides ou d'ester gras insaturés majoritairement présents dans les huiles végétales, par des réactions de métathèse.

La demande de brevet FR 2878 246 au nom du même déposant décrit une réaction de métathèse d'un corps gras insaturé avec de l'éthylène, en présence d'au moins un liquide ionique non-aqueux, qui permet de produire à la fois une fraction oléfinique et une composition d'esters de monoalcool ou de polyol. Appliqué plus particulièrement à une huile de tournesol oléique, à une huile de colza oléique, ou à un mélange d'esters de monoalcool desdites huiles, le procédé permet de produire à la fois une fraction oléfinique et une composition d'esters de monoalcool ou de glycérol dont en général plus de la moitié des chaînes est constituée de chaînes insaturées en C10.

La demande de brevet FR 2 896 500 "Procédé de co-production d'oléfines et de diesters ou de diacides par homométathèse de corps gras insaturés dans des liquides ioniques non-aqueux", au nom du même déposant, décrit un procédé dans lequel on fait réagir, par une réaction d'homométathèse, un corps gras insaturé en présence d'au moins un liquide ionique non-aqueux, qui permet de produire à la fois une fraction oléfinique à longue chaîne, dont la double liaison est en position interne, et une composition de diesters de monoalcool ou diacides. Appliqué plus particulièrement à un mélange d'ester d'une huile de tournesol oléique ou d'une huile de colza oléique, le procédé permet de produire à la fois une fraction oléfinique et une composition de diesters de monoalcool ou de diacides dont en général plus de la moitié des chaînes est constituée de chaînes insaturées en C18.

La publication de Warwel et al. (Chemosphere 43 (2001) 39-48) décrit l'éthénolyse d'esters d'acide gras issus d'huiles de tournesol permettant d'obtenir du décène-1 et du 9-décénoate de méthyle. D'autre part, il décrit la dimérisation d'esters méthyliques d'acides gras insaturés, tels que le 9-décénoate de méthyle, pour former des acides dicarboxyliques à longue chaîne (C18) par réaction de métathèse.

### Objet de l'invention

La présente invention propose un procédé de coproduction d'oléfines et de diesters ou de diacides insaturés incluant :
a) une étape de métathèse d'au moins un corps gras insaturé, riche en esters ou acides gras insaturés avec de l'éthylène, en présence d'au moins un liquide ionique non-aqueux, et d'au moins un catalyseur,
b) une étape de séparation et de recyclage du liquide ionique contenant le catalyseur utilisé dans l'étape (a),
c) une étape de séparation des oléfines et des esters ou des acides insaturés produits dans l'étape (a),
d) une étape d'homométathèse de la fraction esters ou acides insaturés produits dans l'étape (a) et séparés dans l'étape (c), réalisée en phase liquide en présence d'au moins un catalyseur et
e) éventuellement, une étape de séparation et de recyclage du liquide ionique contenant le catalyseur, lorsqu'on en utilise dans l'étape (d).

Cette succession d'étapes permet de produire de façon simultanée majoritairement des oléfines dont la double liaison est en position terminale (oléfines alpha) et majoritairement des diesters, ou de diacides insaturés à longues chaînes. Un des intérêts de ce procédé est de réutiliser l'éthylène co-produit dans l'étape (d) du procédé en le recyclant à l'entrée de la première étape de métathèse.

L'invention concerne également une installation utilisée pour mettre en oeuvre le procédé selon l'invention.

### Description des dessins

La Figure 1 représente un schéma de l'installation permettant de mettre en oeuvre le procédé selon l'invention.

La Figure 2 représente un schéma de l'installation permettant de mettre en oeuvre le procédé selon l'invention comprenant une étape de recyclage du liquide ionique utilisé dans la réaction d'homométathèse.

### Description détaillée de l'invention

Les charges traitées dans le procédé selon l'invention, sont des corps gras insaturés comprenant au moins un monoacide carboxylique possédant de 12 à 22 atomes de carbone et comportant au moins une insaturation éthylénique et/ou un monoester formé entre un tel monoacide et au moins un composé aliphatique saturé monohydroxylé (monoalcool), le monoalcool étant par exemple un monoalcool de 1 à 8 atomes de carbone.

Plus particulièrement, le procédé de l'invention peut s'appliquer à l'acide oléique, acide gras dont la chaîne est porteuse d'une seule insaturation, ou à ses esters dérivés. Dans ce cas, le procédé conduit à la formation de seulement deux produits, le décène-1 et le diacide-1,18 du 9-octadécène ou le diester correspondant.

Cependant, il n'existe pas dans la nature de corps gras d'origine végétale ou animale dont les chaînes grasses seraient exclusivement constituées de chaînes oléiques. L'obtention d'un ester de l'acide oléique pur nécessite donc de recourir à une opération de séparation et de purification faisant le plus souvent appel à la distillation dans des conditions délicates et donc coûteuses.

La nature des produits obtenus, ainsi que leur quantité dépendront donc de la composition en acides gras (nature et abondance) de la matière première grasse utilisée.

L'obtention de produits riches en décène-1 implique l'utilisation d'une matière première riche en esters de l'acide oléique.

Ces huiles sont préférentiellement utilisées sous la forme d'un mélange d'esters de monoalcool, comme par exemple le méthanol, l'éthanol, le propanol ou plus généralement tout monoalcool renfermant de 1 à 8 atomes de carbone.

Les esters d'acides gras saturés présents dans l'huile de tournesol oléique ou l'huile de colza oléique ou dans les esters de monoalcools de ces huiles ne sont pas réactifs dans les réactions de métathèse et sont retrouvés à la fin de l'opération.

L'huile végétale considérée (ou l'ester de monoalcool de cette huile) est choisie, de façon préférée, parmi l'huile de tournesol oléique ou l'huile de colza oléique (ou les esters de monoalcool de ces huiles). Ces huiles particulières et les esters de monoalcools dérivés de ces huiles sont caractérisés par leur composition en acides gras, notamment par la nature et la proportion de leurs acides gras insaturés. Dans ces huiles ou dans les esters de monoalcool de ces huiles, en général au moins 80 % des chaînes d'acides gras sont constituées de chaînes oléiques (C18), la teneur en chaînes grasses linoléiques n'excède pas 12 % et la teneur en chaînes grasses linoléniques n'excède pas 0,3 %. Aucune autre chaîne oléfinique n'est présente dans ces huiles ou dans les esters de monoalcool de ces huiles à une teneur supérieure à 0,3 %, tandis que la teneur en chaînes saturées par exemple palmitique ou stéarique est comprise entre 5 % et 15 %.

On peut considérer par exemple un ester méthylique d'une huile de tournesol oléique dont la composition est la suivante :
- oléate de méthyle : environ 83 % en masse
- linoléate de méthyle : environ 10 % en masse
- palmitate de méthyle : environ 3 % en masse
- stéarate de méthyle : environ 4 % en masse.

On décrit ci-après plus en détails les conditions particulières des différentes étapes du procédé selon l'invention, réalisé à partir d'un corps gras contenant au moins une insaturation éthylénique, et ayant pour objectif de produire simultanément une fraction oléfinique, composée majoritairement d'oléfines alpha, et un diester ou un diacide à longue chaîne insaturée.

L'objet principal de **la première étape (a)** est de transformer l'ester ou l'acide gras insaturé dérivé d'une huile végétale, notamment un ester méthylique d'une huile de tournesol oléique dont la composition a été détaillée ci-dessus
- d'une part, en une fraction oléfinique majoritaire en décène-1 (fraction A) ;
- et d'autre part, en un mélange d'esters dont plus de la moitié des chaînes sera constituée de chaînes insaturées en C10 (fraction B). Une telle composition ne correspond à aucun corps gras connu. Un tel mélange d'esters est caractéristique en ce que sa concentration en chaînes insaturées en C10 est très élevée. Il est caractérisé également par la position de l'insaturation située entre l'atome de carbone en position 9 et celui en position 10 sur la chaîne carbonée. Cette position de l'insaturation est différente de celle observée dans les produits naturels.

Cette première étape implique une réaction de métathèse de corps gras insaturés, du type de ceux décrits précédemment, avec un excès d'éthylène. Elle est réalisée en présence d'au moins un catalyseur et en présence d'au moins un liquide ionique non-aqueux selon les enseignements de la demande de brevet français N° 04/12414 au nom de la demanderesse, citée plus haut.

Dans cette étape, le catalyseur à base de complexe du ruthénium est avantageusement mis en oeuvre dans le liquide ionique non-aqueux, dans lequel les produits formés sont très peu miscibles. Le catalyseur reste immobilisé et stabilisé dans le liquide ionique. Ce dernier contenant le catalyseur peut être recyclé et réutilisé.

Les catalyseurs utilisés dans cette première étape du procédé comprenant la réaction de métathèse des corps gras insaturés avec de l'éthylène en excès sont des catalyseurs comprenant au moins un composé du ruthénium.

Le solvant ionique non-aqueux est choisi dans le groupe formé par les sels liquides qui ont pour formule générale Q⁺ A⁻ dans laquelle Q⁺ représente un ammonium quaternaire, un phosphonium quaternaire, un guanidinium quaternaire ou un sulfonium quaternaire et A- représente tout anion susceptible de former un sel liquide à basse température, c'est-à-dire en dessous de 90°C et avantageusement d'au plus 85°C, et de préférence en dessous de 50°C.

Selon le procédé de l'invention, la réaction de métathèse selon l'étape (a) du procédé est réalisée avec de l'éthylène utilisé en excès. La réaction peut être conduite en l'absence ou en présence d'un co-solvant organique. Dans le cas où un solvant ou un mélange de solvants est utilisé, son rôle peut être d'améliorer la solubilisation des réactifs et du catalyseur dans le liquide ionique. Il peut aussi servir à optimiser l'extraction des produits dans une deuxième phase. Parmi les solvants envisageables, on peut citer par exemple les chloroalcanes, tels que le dichlorométhane, le chloroforme ou les dichloro- ou trichloroéthane, les solvants aromatiques tels que le toluène, les xylènes ou le chlorobenzène ou aliphatiques tels que l'heptane ou le cyclohexane.

La réaction de métathèse du corps gras insaturé (monoacide ou monoester) par exemple dérivé de l'huile de tournesol oléique ou de l'huile de colza oléique avec de l'éthylène, utilisé en excès, peut être conduite en système fermé (batch), en système semi-ouvert ou en continu avec un ou plusieurs étages de réaction.

Une vigoureuse agitation doit assurer un bon contact entre les réactifs (gazeux et liquide) et le mélange catalytique. La température de réaction peut être de 0°C à +150°C, de préférence de 20°C à 120°C.

On peut opérer au-dessus ou en dessous de la température de fusion du milieu, l'état solide dispersé n'étant pas une limitation au bon déroulement de la réaction.

La pression peut aller par exemple de la pression atmosphérique à 50 MPa.

L'éthylène peut être utilisé pur ou en mélange ou dilué avec une paraffine (inerte).

**La deuxième étape (b)** du procédé selon l'invention consiste à séparer la phase liquide ionique contenant le catalyseur, de la phase contenant les produits de la réaction de métathèse réalisée dans la première étape. Dans cette 2^{ème} étape, les produits de la réaction issus de la première étape de métathèse peuvent être séparés du liquide ionique contenant le catalyseur soit par distillation du fait de la non-volatilité du liquide ionique, soit par décantation du fait de la faible solubilité des oléfines formées dans le liquide ionique.

**La troisième étape (c)** selon le procédé de la présente invention consiste à séparer les produits formés lors de la première étape de métathèse du procédé selon l'invention. La fraction purement oléfinique (fraction A constituée de mono- et dioléfines, majoritairement composée de décène-1) peut être aisément séparée de la fraction B constituée d'un mélange d'esters (ou d'acides) présents (majoritairement le décénoate de méthyle) par une étape d'évaporation, compte tenu de la différence de point d'ébullition de ces deux fractions.

Dans le procédé selon la présente invention, on peut ensuite soumettre le mélange d'oléfines isolé précédemment (fraction A) à une distillation visant à séparer les dioléfines et les monooléfines, ainsi que tout excès d'éthylène. L'éthylène en excès peut être recyclé vers l'étape (a) du même procédé ou être utilisé lors d'une nouvelle réaction de métathèse, tandis que chacune des autres mono- (ou di-) oléfines peut être valorisée et utilisée séparément.

Après évaporation de la fraction A purement oléfinique, le milieu réactionnel résiduel (fraction B) contient en conséquence un mélange d'esters, c'est-à-dire un ester de l'acide 9-décènoique sous la forme ester de monoalcool, ou sous forme d'acide et aussi éventuellement les acides ou les esters des acides saturés présents dans la matière première, c'est-à-dire les acides palmitique et stéarique sous la forme esters de monoalcool ou sous forme d'acide selon la matière première utilisée. Ces structures saturées ne sont pas concernées par la réaction de métathèse.

**La quatrième étape (d)** du procédé selon l'invention, consiste à soumettre la fraction B constituée d'esters ou d'acides insaturés à une réaction d'homométathèse.

Les catalyseurs utilisés dans cette quatrième étape du procédé pour effectuer la réaction d'homométathèse des esters ou acides insaturés sont des catalyseurs comprenant au moins un composé du ruthénium.

Cette réaction d'homométathèse est effectuée en phase liquide. On peut envisager de mettre en oeuvre le catalyseur dans un liquide ionique non-aqueux tel que décrit selon l'étape (a) du procédé.

On peut également envisager de conduire la réaction en l'absence de solvant ou en présence d'un solvant organique. Parmi les solvants envisageables pour l'invention, on peut citer par exemple les chloroalcanes, tels que le dichlorométhane, le chloroforme ou les dichloro- ou trichloroéthane, les solvants aromatiques tels que le toluène, les xylènes, ou le chlorobenzène, ou aliphatiques tels que l'heptane ou le cyclohexane.

La température de réaction peut être de 0°C à +150°C, de préférence de 20°C à 120°C.

La réaction de métathèse, selon cette quatrième étape du procédé, peut être conduite en système en système semi-ouvert ou en continu avec un ou plusieurs étages de réaction.

L'éthylène gazeux co-produit dans cette étape est séparé et transféré vers l'étape (a) du procédé de l'invention.

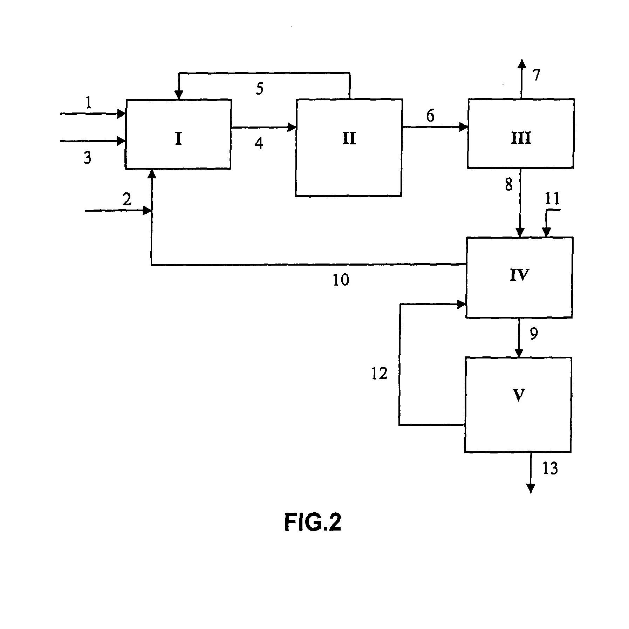

Dans le cas où la réaction d'homométathèse réalisée dans l'étape (d) utilise un liquide ionique comme solvant du catalyseur, le procédé selon l'invention peut comporter **une cinquième étape (e)** qui consiste à séparer le liquide ionique contenant le catalyseur des produits de la réaction et à recycler le liquide ionique à l'entrée de l'étape (d) de métathèse de la même façon qu'après l'étape (b) (illustré par la Figure 2).

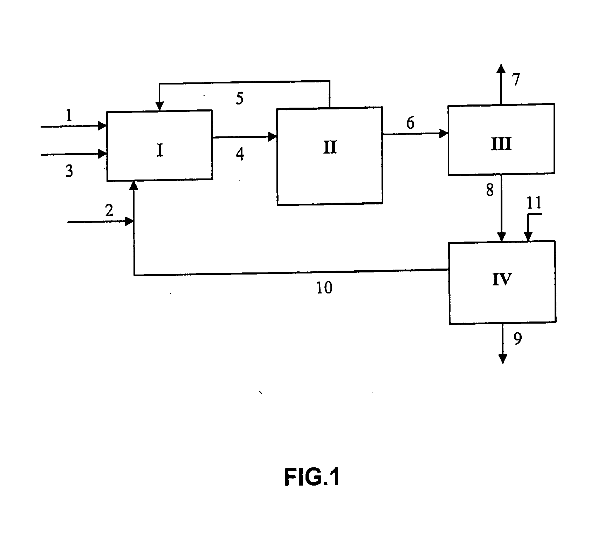

L'invention concerne également une installation (illustrée par les Figures 1 et 2) utilisée pour mettre en oeuvre le procédé décrit ci-dessus.

Elle est constituée de différentes zones dans lesquelles ont lieu successivement les différentes étapes du procédé selon l'invention :
- une zone I de métathèse de corps gras insaturés avec un excès d'éthylène, ladite zone 1 comportant au moins un moyen 1 pour l'introduction du corps gras insaturé, au moins un moyen 2 pour l'introduction de l'éthylène, et au moins un moyen 3 pour l'introduction du catalyseur. La sortie de l'effluent depuis cette zone I est effectuée par l'intermédiaire d'au moins un moyen 4 ;
- une zone II dans laquelle a lieu le recyclage du liquide ionique. Dans cette zone, on sépare le liquide ionique contenant le catalyseur de l'effluent issu de la zone I, ladite zone comportant au moins un moyen 4 pour l'introduction de l'effluent issu de la zone I, au moins un moyen 5 pour le recyclage du liquide ionique contenant le catalyseur à l'entrée de la zone I, au moins un moyen 6 pour la sortie de la phase organique contenant la fraction oléfinique et la fraction ester ou acide gras insaturée ;
- une zone III de séparation de l'effluent organique issu de la zone II, comportant au moins un moyen 6 pour l'introduction de l'effluent issu de la zone II, au moins un moyen 7 pour la sortie de la fraction oléfinique A, et au moins un moyen 8 pour la sortie de la fraction B constituée d'esters ou d'acides gras insaturés ;
- une zone IV dans laquelle a lieu la réaction d'homométathèse comprenant au moins un moyen 8 pour l'introduction de l'effluent à convertir issu de la zone III, au moins un moyen 9 pour la sortie de l'effluent contenant principalement le diester ou le diacide insaturé à longue chaîne formé, au moins un moyen 10 pour séparer l'éthylène gazeux co-produit par la réaction d'homométathèse, le moyen 10 de séparation de l'éthylène dans cette zone IV étant relié au moyen 2 d'introduction de l'éthylène dans la zone I, et au moins un moyen 11 d'introduction du catalyseur,

Dans le cas où la réaction de métathèse de la zone IV est réalisée en présence d'un liquide ionique, le procédé selon l'invention peut éventuellement comporter :
- une zone V de séparation du liquide ionique contenant le catalyseur de l'effluent formé, comportant au moins un moyen 12 pour séparer le liquide ionique et le recycler à l'entrée de la zone IV, et au moins un moyen 13 pour séparer l'effluent formé dans cette zone (illustrée dans la Figure 2).
Les exemples suivants illustrent l'invention sans la limiter.

### Exemple 1 : Métathèse par éthénolyse de l'oléate de méthyle catalysée par un complexe de type 3 (Figure 1) dans un liquide ionique

Dans un réacteur autoclave équipé d'un système d'agitation et d'un capteur de pression, on introduit sous atmosphère inerte d'argon 1 mL de liquide ionique butyl-3-diméthyl-1,2-imidazolium bis-triflylamidure de formule : [BMMI]⁺ [N(CF₃SO₂)₂]⁻ préséché sous vide une nuit à 80°C, 148 mg d'oléate de méthyle (origine Fluka, de pureté supérieure à 98 %), et 15 mg de complexe de formule :

Cl₂Ru(=CH-*o*-O-iPrC₆H₄)PCy₃

(synthétisé par réaction du complexe de Grubbs 1^{ère} génération de formule : Cl₂Ru(=CHC₆H₅)(PCy₃)₂ avec le 1-isopropoxy-2-vinylbenzène en présence de CuCl), soit 5 % molaire de catalyseur par rapport à l'oléate de méthyle. L'autoclave est ensuite tiré sous vide puis pressurisé jusqu'à obtention d'une pression de 10 bar (1 MPa) d'éthylène (origine Alphagaz, qualité N25). La température est maintenue constante à 20°C.

Le milieu est agité à température ambiante pendant 2 heures, puis l'excès d'éthylène est lentement chassé par retour à la pression atmosphérique, à une température n'excédant pas 20°C et l'autoclave est remis sous atmosphère d'argon. Les produits sont séparés du liquide ionique par addition de 2 à 3 mL d'heptane distillé sur CaH₂ et dégazé. Un aliquote (100 µL) de la solution extraite est passé sur une courte colonne de silice (2 cm) éluée à l'éther diéthylique. Il est analysé par chromatographie en phase gaz (colonne ZB-1, 100 % diméthylpolysiloxane, 30 mètres, gaz vecteur hélium 2 mL/min, programmation de température : 60°C puis 5°C/min jusqu'à 220°C) couplée à un spectromètre de masse.

La conversion de l'oléate de méthyle est de 95 %. Elle est calculée en utilisant le décane comme étalon interne. Les produits de la réaction sont composés de décène-1 (fraction A) et de décénoate de méthyle (fraction B).

On ne détecte pas la présence d'isomères du décène-1. Les produits d'homométathèse sont présents à l'état de traces, non quantifiables.

### Exemple 2 : Recyclage du liquide ionique contenant le catalyseur

Après le premier cycle réalisé selon l'Exemple 1, l'autoclave contenant le liquide ionique et le catalyseur est tiré sous vide afin d'éliminer les traces d'heptane. Sous atmosphère d'argon, on rajoute 148 mg d'oléate de méthyle, puis on pressurise le réacteur jusqu'à obtention d'une pression de 10 bar (1 MPa) d'éthylène. On maintient la température à 20°C.

On réalise alors la même procédure que celle décrite dans l'Exemple 1 pour analyser les produits formés.

On réalise ainsi 3 cycles successifs sans rajouter de catalyseur ni de liquide ionique.

On détermine à chaque cycle la conversion de l'oléate de méthyle et la composition des produits formés (Tableau 1 ci-dessous).

**Tableau 1**

| | Conversion oléate de méthyle (% poids) | Produits formés |
|---|---|---|
| 1^{er} cycle (Exemple 1) | 95 | Décène-1 + décénoate de méthyle |
| 2^{ème} cycle | 95 | Décène-1 + décénoate de méthyle |
| 3^{ème} cycle | 85 | Décène-1 + décénoate de méthyle |

### Exemple 3 : Homométathèse du décénoate de méthyle

Dans un récipient en verre, on ajoute 50 mg (0,062 mmole soit 0,01 éq.) de complexe de ruthénium (1,3-Bis-(2,4,6-trimethylphenyl)-2-imidazolidinylidene) dichloro(benzylidene)(tricyclohexylphosphine) ruthénium, 1,5 mL (6,62 mmol soit 1 éq) de décénoate de méthyle (fraction B) obtenu après avoir séparé les deux fractions produites selon l'Exemple 1 et 1mL de bis(trifluoromethanesulfonyl)amidure de1-butyl-1-methylpyrrolidinium de formule (BMPyrr)(NTf2), 2mL d'heptane ainsi que 0,1 mL de dodécane (étalon interne pour la chromatographie).

Le mélange est biphasique. Il est agité et maintenu à température ambiante. Au bout d'un temps de réaction de deux heures, un aliquote de la phase liquide supérieure est extraite pour des analyses de chromatographie en phase gazeuse. Les analyses indiquent que la réaction de métathèse a lieu de façon satisfaisante, conduisant à la formation de diméthyloctadecene-1,18-dioate.

La conversion du décénoate de méthyle est de 70% en poids.

## Revendications

1. Procédé pour produire à la fois une fraction oléfinique et une composition de diesters ou de diacides à partir de corps gras insaturés **caractérisé en ce qu'**il comprend une succession d'étapes incluant ;
a) une étape de mise en contact dans des conditions de métathèse, d'au moins un corps gras insaturé comprenant au moins un monoacide carboxylique possédant de 12 à 22 atomes de carbone et comportant au moins une insaturation éthylénique ou un monoester d'un tel monoacide, avec de l'éthylène en excès, en présence d'un catalyseur et en présence d'au moins un liquide ionique non-aqueux, produisant conjointement une fraction oléfinique et une fraction monoesters ou monoacides de corps gras insaturés,
b) une étape de séparation et de recyclage du liquide ionique contenant le catalyseur utilisé dans l'étape (a),
c) une étape de séparation de la fraction oléfinique (fraction A) et de la fraction monoesters ou monoacides de corps gras insaturés (fraction B) produits dans l'étape (a),
d) une étape de réaction d'homométathèse de la fraction B de monoesters ou monoacides de corps gras insaturés séparée dans l'étape (c), ladite réaction étant réalisée en phase liquide en présence d'un catalyseur, l'éthylène gazeux co-produit lors de cette étape étant séparé et transféré vers l'étape a)
et **en ce qu'**on utilise comme catalyseur au moins un composé du ruthénium dans les étapes (a) et (d).

2. Procédé selon la revendication 1 **caractérisé en ce qu'**il met en jeu à l'étape (a) une charge constituée d'un corps gras insaturé comprenant au moins un ester formé entre au moins un monoacide carboxylique comportant au moins une insaturation éthylénique et possédant de 12 à 22 atomes de carbone et au moins un composé aliphatique monohydroxylé (monoalcool) comportant de 1 à 8 atomes de carbone.

3. Procédé selon la revendication 1 ou 2 **caractérisé en ce qu'**il met en jeu à l'étape (a) un corps gras insaturé choisi parmi les mélanges d'esters de monoalcool des huiles de tournesol oléiques et des huiles de colza oléiques, pour produire à la fois une fraction oléfinique A et une composition d'esters de monoalcool dont au moins une partie des chaînes est constituée de chaînes insaturées en C10 (fraction B).

4. Procédé selon la revendication 3 **caractérisé en ce que** la charge introduite dans l'étape (a) est un ester méthylique d'une huile de tournesol oléique dont la composition est la suivante :
- oléate de méthyle : environ 83 % en masse
- linoléate de méthyle : environ 14 % en masse
- palmitate de méthyle : environ 3 % en masse
- stéarate de méthyle : environ 4 % en masse

5. Procédé selon la revendication 4 **caractérisé en ce que** la fraction A obtenue est majoritaire en décène-1 et que la fraction B est majoritairement du 9-décénoate de méthyle.

6. Procédé selon l'une des revendications 1 à 5 **caractérisé en ce que** le liquide ionique non-aqueux est choisi dans le groupe formé par les sels liquides de formule générale Q+ A- dans laquelle Q+ représente un phosphonium quaternaire, un ammonium quaternaire, un guanidinium quaternaire ou un sulfonium quaternaire et A- représente tout anion susceptible de former un sel liquide en dessous de 90°C.

7. Procédé selon l'une des revendications 1 à 6 **caractérisé en ce que** l'étape (b) est une étape de séparation de la phase liquide ionique contenant le catalyseur et des produits de réaction issus de l'étape (a) soit par distillation soit par décantation.

8. Procédé selon l'une des revendications 1 à 7 **caractérisé en ce qu'**il comprend, au cours de l'étape (c), une étape de séparation des fractions A et B par évaporation.

9. Procédé selon la revendication 8 **caractérisé en ce qu'**il comprend en outre une étape dans laquelle les monooléfines et les dioléfines de la fraction A sont séparées par distillation.

10. Procédé selon l'une des revendications 3 à 9 **caractérisé en ce que** la charge introduite à l'étape (d) est un ester de l'acide 9-décènoique sous la forme ester de monoalcool.

11. Procédé selon l'une des revendications 1 à 10 **caractérisé en que** l'homométathèse de l'étape (d) est réalisée en présence d'un liquide ionique non aqueux contenant le catalyseur.

12. Procédé selon la revendication 11 **caractérisé en ce que** le procédé comprend en outre une étape de séparation et de recyclage du liquide ionique utilisé à l'étape (d) et contenant le catalyseur.

13. Installation permettant de mettre en oeuvre le procédé selon les revendications 1 à 12 constituée des zones I à V suivantes :
• une zone I de métathèse comportant au moins un moyen 1 pour l'introduction du corps gras insaturé, au moins un moyen 2 pour l'introduction de l'éthylène, et au moins un moyen 3 pour l'introduction du catalyseur, la sortie de l'effluent depuis cette zone I s'effectuant par l'intermédiaire d'au moins un moyen 4,
• une zone Il dans laquelle a lieu la séparation et le recyclage du liquide ionique contenant le catalyseur de l'effluent issu de la zone I, ladite zone comportant au moins un moyen 4 pour l'introduction de l'effluent issu de la zone I, au moins un moyen 5 pour le recyclage du liquide ionique contenant le catalyseur à l'entrée de la zone I, au moins un moyen 6 pour la sortie de la phase organique contenant la fraction oléfinique et la fraction ester ou acide gras insaturés,
• une zone III de séparation comportant au moins un moyen 6 pour l'introduction de l'effluent issu de la zone II, au moins un moyen 7 pour la sortie de la fraction oléfinique A, et au moins un moyen 8 pour la sortie de la fraction B constituée d'esters ou acides gras insaturés,
• une zone IV d'homométathése comprenant au moins un moyen 8 pour l'introduction de l'effluent à convertir issu de la zone III, au moins un moyen 9 pour la sortie de l'effluent contenant principalement le diester ou le diacide insaturé à longue chaîne formé, au moins un moyen 10 pour séparer l'éthylène co-produit par la réaction d'homométathèse, le moyen 10 de séparation de l'éthylène dans cette zone IV étant relié au moyen 2 d'introduction de l'éthylène dans la zone I, au moins un moyen 11 d'introduction du catalyseur.

14. Installation selon la revendication 13 **caractérisée en ce qu'**elle comprend en outre une zone V de séparation du liquide ionique contenant le catalyseur de l'effluent formé, comportant au moins un moyen 12 pour séparer le liquide ionique et le recycler à l'entrée de la zone IV, et au moins un moyen 13 pour séparer l'effluent formé dans cette zone.

## Claims

1. A process for producing both an olefinic fraction and a composition of diesters or diacids from unsaturated fats, **characterized in that** it comprises a succession of steps including:
a) a step for bringing into contact, under methathesis conditions, at least one unsaturated fat comprising at least one carboxylic monoacid containing 12 to 22 carbon atoms and comprising at least one ethylenically unsaturated bond or a monoester of said monoacid, with excess ethylene, in the presence of a catalyst and in the presence of at least one non-aqueous ionic liquid, jointly producing an olefinic fraction and a fraction of monoesters or monoacids of unsaturated fats;
b) a step for separating and recycling ionic liquid containing the catalyst used in step a);
c) a step for separating the olefinic fraction (fraction A) and the unsaturated fat monoester or monoacid fraction (fraction B) produced in step a);
d) a step for homometathesis of fraction B of unsaturated fat monoesters or monoacids separated in step c), said reaction being carried out in the liquid phase in the presence of one catalyst, co-produced gaseous ethylene being separated and transferred to step a),
and **in that** at least one ruthenium compound is used as catalyst in steps a) and d).

2. A process according to claim 1, **characterized in that** in step a) it employs a feed constituted by an unsaturated fat comprising at least one ester formed between at least one carboxylic monoacid comprising at least one ethylenically unsaturated bond and containing 12 to 22 carbon atoms and at least one monohydroxylated aliphatic compound (mono-alcohol) containing 1 to 8 carbon atoms.

3. A process according to claim 1 or claim 2, **characterized in that** in step a) it employs an unsaturated fat selected from mixtures of mono-alcohol esters of oleic sunflower seed oils and oleic rapeseed oils to produce both an olefinic fraction A and a composition of mono-alcohol esters at least a portion of the chains of which is constituted by unsaturated C₁₀ chains (fraction B).

4. A process according to claim 3, **characterized in that** the feed introduced in step a) is a methyl ester of an oleic sunflower seed oil with the following composition:
• methyl oleate: about 83% by weight;
• methyl linoleate: about 10% by weight;
• methyl palmitate: about 3% by weight;
• methyl stearate: about 4% by weight.

5. A process according to claim 4, **characterized in that** the fraction A obtained is mainly 1-decene and fraction B is mainly methyl 9-decenoate.

6. A process according to one of claims 1 to 5, **characterized in that** the non-aqueous ionic liquid is selected from the group formed by liquid salts with general formula Q⁺A⁻ in which Q⁺ represents a quaternary phosphonium, a quaternary ammonium, a quaternary guanidinium or a quaternary sulphonium and A⁻ represents any anion which is capable of forming a liquid salt below 90°C.

7. A process according to one of claims 1 to 6, **characterized in that** step b) is a step for separating the ionic liquid phase containing the catalyst and the reaction products from step a) either by distillation or by decanting.

8. A process according to one of claims 1 to 7, **characterized in that** it comprises a step for separating fractions A and B by evaporation during step c).

9. A process according to claim 8, **characterized in that** it further comprises a step in which the mono olefins and the di-olefins of fraction A are separated by distillation.

10. A process according to one of claims 3 to 9, **characterized in that** the feed introduced in step d) is an ester of 9-decenoic acid in the mono-alcohol ester form.

11. A process according to one of claims 1 to 10, **characterized in that** homometathesis of step d) is carried out in the presence of a non-aqueous ionic liquid containing the catalyst.

12. A process according to claim 11, **characterized in that** the process further comprises a step for separating and recycling the ionic liquid used in step d) containing the catalyst.

13. A facility for carrying out the process of claims 1 to 12, constituted by the following zones I to IV:
• a methathesis zone I comprising at least one means 1 for introducing unsaturated fat, at least one means 2 for introducing ethylene, and at least one means 3 for introducing catalyst, the effluent being removed from said zone I via at least one means 4;
• a zone II in which the ionic liquid containing the catalyst is separated from the effluent from zone I and recycled, said zone comprising at least one means 4 for introducing effluent from zone I, at least one means 5 for recycling the ionic liquid containing the catalyst to the inlet to zone I, at least one means 6 for removing the organic phase containing the olefinic fraction and the unsaturated ester or fatty acid fraction;
• a separation zone III comprising at least one means 6 for introducing effluent from zone II, at least one means 7 for removing olefinic fraction A, and at least one means 8 for removing fraction B constituted by unsaturated esters or fatty acids;
• a homometathesis zone IV comprising at least one means 8 for introducing effluent to be converted derived from zone III, at least one means 9 for removing effluent principally containing the unsaturated long chain diester or diacid formed, at least one means 10 for separating the ethylene co-produced by the homometathesis reaction, the means 10 for separating ethylene in said zone IV being connected to the means 2 for introducing ethylene into zone I, and at least one means 11 for introducing catalyst.

14. A facility according to claim 13, **characterized in that** it further comprises a zone V for separating the ionic liquid containing the catalyst from the effluent formed, comprising at least one means 12 for separating the ionic liquid and recycling it to the inlet to zone IV, and at least one means 13 for separating the effluent formed in said zone.

## Patentansprüche

1. Verfahren zum gleichzeitigen Produzieren einer Olefinfraktion und einer Zusammensetzung aus Diestern oder Diaziden aus ungesättigten Fetten, **dadurch gekennzeichnet, dass** es eine Abfolge von Schritten umfasst, die Folgendes beinhalten:
a) einen Schritt des Inkontaktbringens, unter Metathesebedingungen, mindestens eines ungesättigten Fettes, das mindestens eine Monocarbonsäure umfasst, die 12 bis 22 Kohlenstoffatome besitzt und mindestens eine ethylenische Ungesättigtheit umfasst, oder einen Monoester einer solchen Monosäure, mit Ethylen im Überschuss, in Gegenwart eines Katalysators in Gegenwart mindestens einer nicht wässrigen ionischen Flüssigkeit, wodurch gemeinsam eine Olefinfraktion und eine Fraktion aus Monoestern oder Monosäuren der ungesättigten Fette produziert wird,
b) einen Schritt der Abscheidung und des Recycelns der ionischen Flüssigkeit, die den Katalysator enthält, die in Schritt (a) verwendet wurde,
c) einen Schritt der Abscheidung der Olefinfraktion (Fraktion A) und der Fraktion aus Monoestern oder Monosäuren der ungesättigten Fette (Fraktion B), die in Schritt (a) produziert wurden,
d) einen Schritt der Homometathesereaktion der Fraktion B aus Monoestern oder Monosäuren der ungesättigten Fette, die in Schritt (c) abgeschieden wurde, wobei die Reaktion in flüssiger Phase in Gegenwart mindestens eines Katalysators ausgeführt wird, wobei das gasförmige Ethylen, das während dieses Schritts coproduziert wurde, abgeschieden wird und zu Schritt a) übertragen wird,
und **dadurch**, dass in den Schritten (a) und (d) als Katalysator mindestens eine Rutheniumverbindung verwendet wird.

2. Verfahren nach Anspruch 1, **dadurch gekennzeichnet, dass** in Schritt (a) eine Beschickung eingesetzt wird, die aus einem ungesättigten Fett besteht, das mindestens einen Ester umfasst, der mindestens zwischen einer Monocarbonsäure, die mindestens eine ethylenische Ungesättigtheit umfasst und 12 bis 22 Kohlenstoffatome besitzt und mindestens einer monohydroxylierten aliphatischen Verbindung (Mönoalkohol), die 1 bis 8 Kohlenstoffatome umfasst, gebildet wird.

3. Verfahren nach Anspruch 1 oder 2, **dadurch gekennzeichnet, dass** in Schritt (a) ein ungesättigtes Fett eingesetzt wird, ausgewählt aus den Gemischen der Monoalkoholester der ölsäurehaltigen Sonnenblumenöle und der ölsäurehaltigen Rapsöle, um gleichzeitig eine Olefinfraktion A und eine Zusammensetzung aus Monoalkoholestern zu produzieren, bei denen mindestens ein Teil der Ketten aus Ketten besteht, die an C10 ungesättigt sind (Fraktion B).

4. Vorfahren nach Anspruch 3, **dadurch gekennzeichnet, dass** die Beschickung, die in Schritt (a) eingeführt wird, ein Methylester eines ölsäurehaltigen Sonnenblumenöls ist, der folgendermaßen zusanunengesetzt ist:
- Methyloleat: etwa 83 Gew.-%
- Methylinoleat: etwa 10 Gew.-%
- Methylpalmitat: etwa 3 Gew.-%
- Methylstearat: etwa 4 Gew.-%

5. Verfahren nach Anspruch 4, **dadurch gekennzeichnet, dass** die erhaltene Fraktion A überwiegend aus 1-Decen besteht und die Fraktion B überwiegend aus Methyl-9-decenoat besteht.

6. Verfahren nach einem der Ansprüche 1 bis 5, **dadurch gekennzeichnet, dass** die nicht wässrige ionische Flüssigkeit aus der Gruppe, gebildet aus den flüssigen Salzen mit der allgemeinen Formel Q⁺A⁻ ausgewählt ist, worin Q⁺ für ein quartäres Phosphcnium, ein quartäres Ammonium, ein quartäres Guanidinium oder ein quartäres Sulfonium steht und A⁻ für jedes Anion steht, das in der Lage ist, unterhalb von 90 °C ein flüssiges Salz zu bilden.

7. Verfahren nach einem der Ansprüche 1 bis 6, **dadurch gekennzeichnet, dass** der Schritt (b) ein Schritt der Abscheidung der ionischen flüssigen Phase ist, die den Katalysator enthält, und der Reaktionsprodukte, die aus dem Schritt (a) stammen, entweder durch Destillation oder durch Dekantierung.

8. Verfahren nach einem der Ansprüche 1 bis 7, **dadurch gekennzeichnet, dass** es im Verlauf von Schritt (c), einen Schritt der Abscheidung der Fraktionen A und B durch Verdampfung umfasst.

9. Verfahren nach Anspruch 8, **dadurch gekennzeichnet, dass** es ferner einen Schritt umfasst, bei dem die Monoolefine und die Diolefine der Fraktion A durch Destillation abgeschieden werden.

10. Verfahren nach einem der Ansprüche 3 bis 9, **dadurch gekennzeichnet, dass** die Beschickung, die in Schritt (d) eingeführt wird, ein Ester der 9-Decensäure in Form eines Monoalkoholesters ist.

11. Verfahren nach einem der Ansprüche 1 bis 10, **dadurch gekennzeichnet, dass** die Homometathese des Schritts (d) in Gegenwart einer nicht wässrigen ionischen Flüssigkeit, die den Katalysator enthält, ausgeführt wird.

12. Verfahren nach Anspruch 11, **dadurch gekennzeichnet, dass** das Verfahren ferner einen Schritt des Abschneidens und des Recycelns der ionischen Flüssigkeit, die in Schritt (d) verwendet wurde und die den Katalysator enthält, umfasst.

13. Vorrichtung, die es ermöglicht, das Verfahren nach den Ansprüchen 1 bis 12 durchzuführen, die aus den folgenden Zonen I bis V besteht:
• einer Zone 1 der Metathese, die mindestens ein Mittel 1 zur Einführung des ungesättigten Fettes, mindestens ein Mittel 2 zur Einführung des Ethylens, und mindestens ein Mittel 3 zur Einführung des Katalysators umfasst, wobei der Austritt des Abflusses aus dieser Zone I mittels mindestens eines Mittels 4 erfolgt,
• einer Zone II, in der das Abscheiden und das Recyceln der ionischen Flüssigkeit, die den Katalysator umfasst, aus dem Abfluss, der aus der Zone I stammt, stattfindet, wobei die Zone mindestens ein Mittel 4 zur Einführung das Abflusses, der aus der Zone I stammt, mindestens ein Mittel 5 zum Recyceln der ionischen Flüssigkeit, die den Katalysator enthält, zum Eingang der Zone 1, mindestens ein Mittel 6 für den Austritt der organischen Phase, die die Olefinfraktion und die Fraktion der ungesättigten Ester oder Fette enthält, umfasst,
• einer Zone III der Abscheidung, die mindestens ein Mittel 6 zur Einführung des Abflusses, der aus der Zone II stammt, mindestens ein Mittel 7 für den Austritt der Olefinfraktion A, und mindestens ein Mittel 8 für den Austritt der Fraktion B, die aus ungesättigten Estern oder Fetten besteht, umfasst,
• einer Homomethathesezone IV, die mindestens ein Mittel 8 zur Einführung des umzuwandalnden Abflusses, der aus der Zone III stammt, mindestens ein Mittel 9 für den Austritt des Abflusses, der im Wesentlichen den Diester oder die gebildete langkettige ungesättigte Disäure umfasst, mindestens ein Mittel 10, um das coproduzierte Ethylen durch die Homometathesereaktion abzuscheiden, wobei das Mittel 10 der Abscheidung des Ethylens in dieser Zone. IV mit dem Mittel 2 zur Einführung des Ethylens in die Zone I verbunden ist, mindestens ein Mittel 11 zur Einführung des Katalysators umfasst

14. Vorrichtung nach Anspruch 13, **dadurch gekennzeichnet, dass** sie ferner eine Zone V zur Abscheidung der ionischen Flüssigkeit, die den Katalysator enthält, aus dem gebildeten Abfluss umfasst, die mindestens ein Mittel 12 zum Abscheiden und zum Recyceln der ionischen Flüssigkeit zum Einlass der Zone IV und mindestens ein Mittel 13 zum Abscheiden des Abflusses, der in dieser Zone gebildet wurde, umfasst.
